Europäisches Patentamt

(19) European Patent Office

Office européen des brevets

(11) Publication number: **0 238 313**
**A1**

## EUROPEAN PATENT APPLICATION

(21) Application number: 87302282.6

(22) Date of filing: 17.03.87

(51) Int. Cl.4: **C 12 M 1/00**
**B 01 L 7/00**

(30) Priority: 17.03.86 GB 8606479

(43) Date of publication of application:
23.09.87 Bulletin 87/39

(84) Designated Contracting States:
AT BE CH DE ES FR GB GR IT LI LU NL SE

(71) Applicant: **FLOW LABORATORIES LIMITED**
**P.O. Box 17 Second Avenue, Ind. Estate**
**Irvine Ayrshire Scotland KA12 8NB (GB)**

(72) Inventor: **Freeman, Richard Harry Mark**
**29 Bellevue Road**
**Ayr Scotland (GB)**

(74) Representative: **Brereton, Paul Arthur et al**
**REDDIE & GROSE 16 Theobalds Road**
**London WC1X 8PL (GB)**

(54) Incubator.

(57) An incubator or oven comprises an insulated housing (1), the walls of which are lined with foil track heating elements (32). A temperature sensor (37) controls the supply of electricity to the heating elements (32) according to the temperature in the incubator or oven. The pattern of foil track (61) varies across the area of the heating element (32) so that different parts of the heating element generate heat at different rates.

**Description**

INCUBATOR

This invention relates to an incubator or oven for a heated environment with a substantially uniform temperature throughout. Such incubators are used, for example, for growing culture samples where it is required to maintain the cultures in an environment which is stable with regard to, for example temperature and or atmosphere.

A conventional incubator comprises a housing surrounded by a water jacket. Water can be heated to a controlled temperature by means of heating coils and circulated through the jacket to heat the incubator. This form of incubator is bulky because of the water jacket. The apparatus is expensive and difficult to move because of the weight of water in the jacket.

Incubators are also manufactured which have electrical heating elements but the heat output of such elements is concentrated into a relatively small area of the wall. This tends to create an uneven temperature environment within the chamber compared with either a water-jacketed unit or the present invention.

The present invention provides an incubator or oven comprising an insulated housing, the walls of which are lined with foil track electrical heating elements, and a thermostat for controlling the supply of electricity to the heating elements according to the temperature in the incubator.

Such an incubator provides a cheap and convenient way of creating a stable environment for culture samples. The humidity and/or composition of the atmosphere in the housing may be regulated as well as its temperature. By dispensing with a water jacket the cost and weight of the incubator can be substantially reduced.

The use of a foil track heating element enables faster transfer of heat from the element to the walls of the incubator because of the greater surface area of the element in contact with the walls compared with conventional heating elements. This, in turn, makes the accurate control of temperature more simple and responsive and reduces the possibility of overshooting the desired temperature. It is particularly important to reduce the likelihood of serious overshoot when using the incubator for cell cultures because too high a temperature may kill the cells. Additionally the faster the heat transfer the more quickly the chamber temperature can recover after the door is opened.

An additional advantage of even heat distribution is that there is less tendency for condensation to occur on the chamber walls at the high humidity normally found in this type of incubator chamber.

The use of a foil track heating element makes it possible more easily to vary the heating profile of the heating element, for example to compensate for different heat losses from different parts of the incubator or oven, particularly near the door where the insulation is broken. The heating profile may be varied by varying the width of the foil track or by varying the density of the heating track pattern across the area of the heating element. An advantage of varying the heating profile so as to achieve a uniform temperature in the oven or incubator is that condensation is reduced - particularly when the incubator is humidified.

The foil track heating elements may each be in the form of a sheet consisting of metallic foil strip with a meandering path sandwiched between layers of electrically insulating material, for example of mylar polyester. There may be several alternating layers of insulating material and metallic conductive material. The metallic foil strip is preferably distributed in such a manner so as to assist in attaining a uniform temperature throughout the incubator or oven cavity, such a distribution may require a variation in concentration of metallic foil strip across a particular sheet. The foil track heating elements may be self adhesive for mounting on the inner walls and may also be mounted on the outer door. A separate element sheet may be used for each wall. The door is preferably insulated for example with glass reinforced plastics material. Holes may be provided in the heating element to allow apparatus to be attached to the surface on which the heating element is to be mounted. Preferably the housing is designed so that the sample element can be used on more than one wall in order to achieve economy of manufacture. For the optimum economy all heating elements should be the same.

The temperature of the incubator or oven may be displayed on a control panel. Control panels may be used to maintain the housing cavity at a set temperature. A common operating temperature for incubators is 37°C but higher temperatures may be used in an oven or for example temperatures up to about 130° for sterilization of an incubator.

A fan mounted on one of the inner walls may be used to circulate the air in the incubator.

A humidity reservoir may be provided in the base of the incubator compartment.

One or more gas inlets may be provided through the housing, for example for $CO_2$, to help maintain a stable environment. The level of a given gas and temperature may be monitored using sensors set on the inside of the compartment. It is preferable to have a good airtight seal on the doors especially if the cultures are to be kept, for example, in a $CO_2$ atmosphere.

An embodiment of the invention is described below with reference to the drawings.

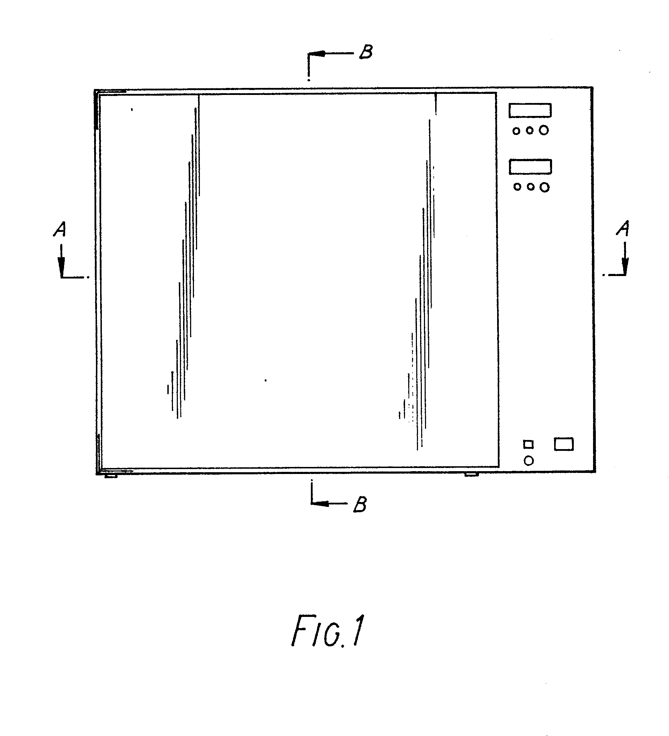

Fig. 1 shows a front elevation of the apparatus in accordance with the invention;

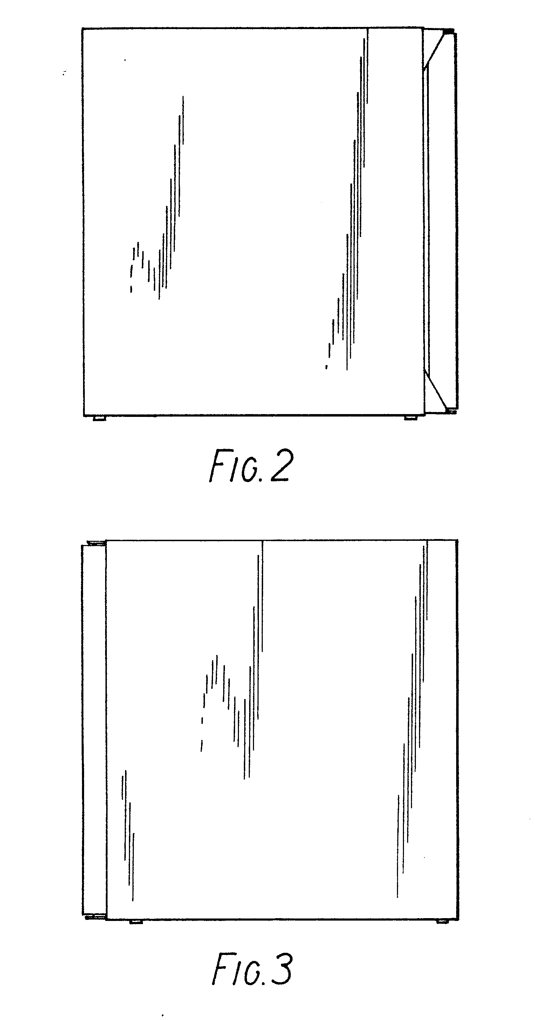

Fig. 2 is a left hand side elevation;

Fig. 3 is a right hand side elevation;

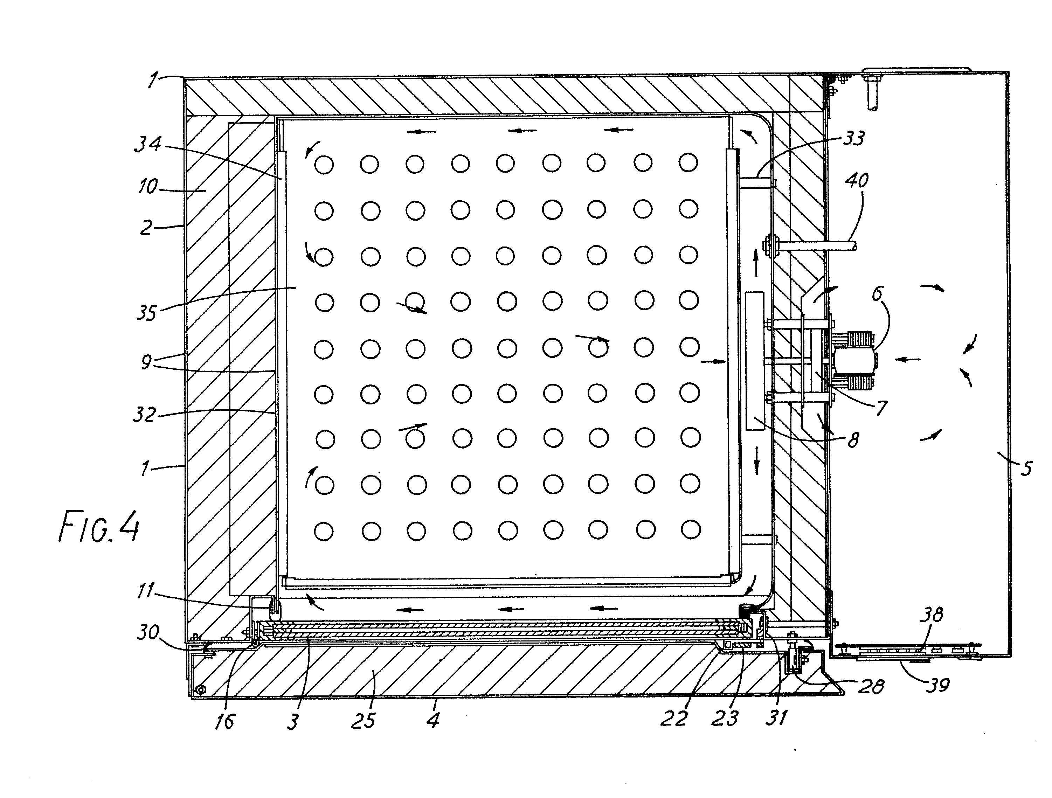

Fig. 4 is a cross-section on the line A-A of Fig. 1 on an enlarged scale;

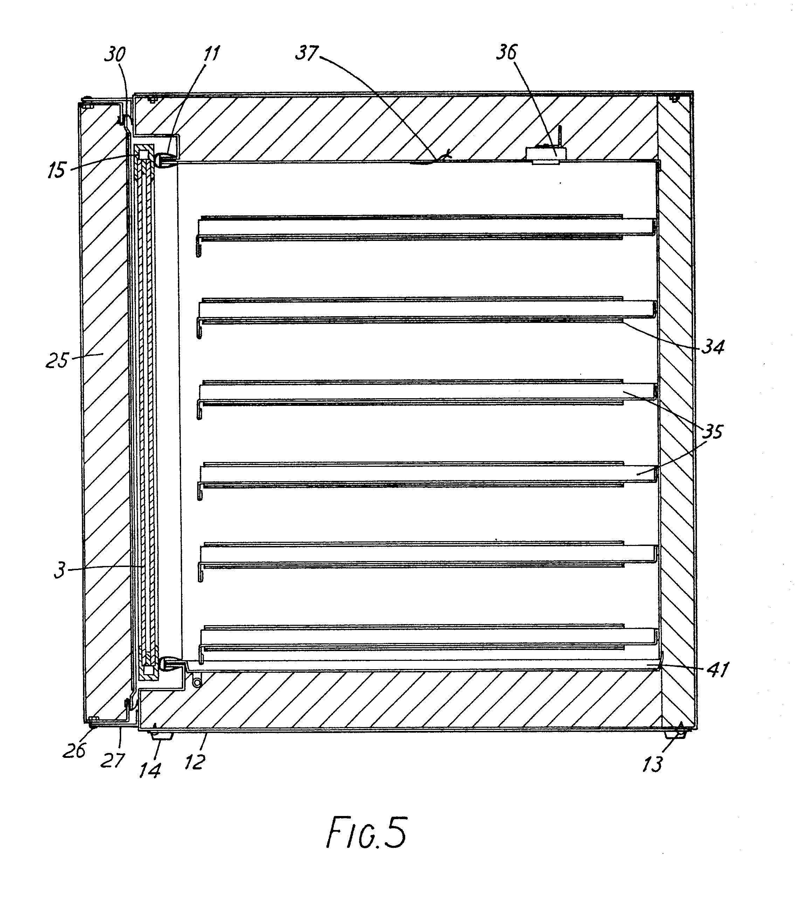

Fig. 5 is a cross-section on the line B-B of Fig. 1 on an enlarged scale;

Fig. 6 is a plan of the foil track heating element designed for use in the apparatus.

The apparatus comprises a rectangular housing 1 made up of five insulated walls 2 which form the top, bottom, back and sides of the housing. The sixth wall at the front of the housing is formed by an inner door 3 and an outer insulated door 4. An instrument unit 5 is attached to one of the side walls. A motor 6 connected to two fans 7 and 8 is mounted against an outer wall of the housing within the instrument unit. The fan 7 is outside the housing and serves to cool the motor. The fan 8 is inside the housing and serves to circulate the atmosphere in the housing.

The housing is constructed from two shells 9 of sheet metal pop-rivetted together at their edges. The space between the shells is insulated with slabs of glass fibre insulating material 10. A silicone seal 11 runs around the opening at the front of the housing and is fixed to the shells 9. This silicone seal forms an airtight seal with the inner door 3 when the door is closed.

A base plate 12 is attached by screws 13 to the underside of the housing. Four feet 14 with screw adjustments are secured to the plate 12.

The inner door 3 is made from a 14 mm double glazed unit in an extruded PVC frame 15 and mounted by hinges 16 to the housing. The inner door is provided with a door catch 22, a catch retaining element 23 is mounted on one of the side walls of the housing.

The outer door 4 is constructed from outer and inner shells pop-rivetted together with insulation of glass reinforced plastics material 25 in the space between the two shells. The outer door is mounted on pivots 26 on brackets 27 attached to one of the side walls of the housing compartment. A magnetic catch 28 is provided on the outer shell of the compartment with a corresponding retaining element 29 in the outer door. Silicone seals are held at the junction of the outer and inner shells of the door; these form an airtight seal with the outer shell of the walls when both doors are closed. The outer door is recessed to accommodate the catch and catch retaining element for the inner door.

A proximity switch 31 is provided to the inner door 3 which turns off the motor for the fan when the inner door is opened.

The inner walls of both the housing and the outer door are each covered with foil track heating elements 32. One sheet or blanket of foil track element is used for each wall.

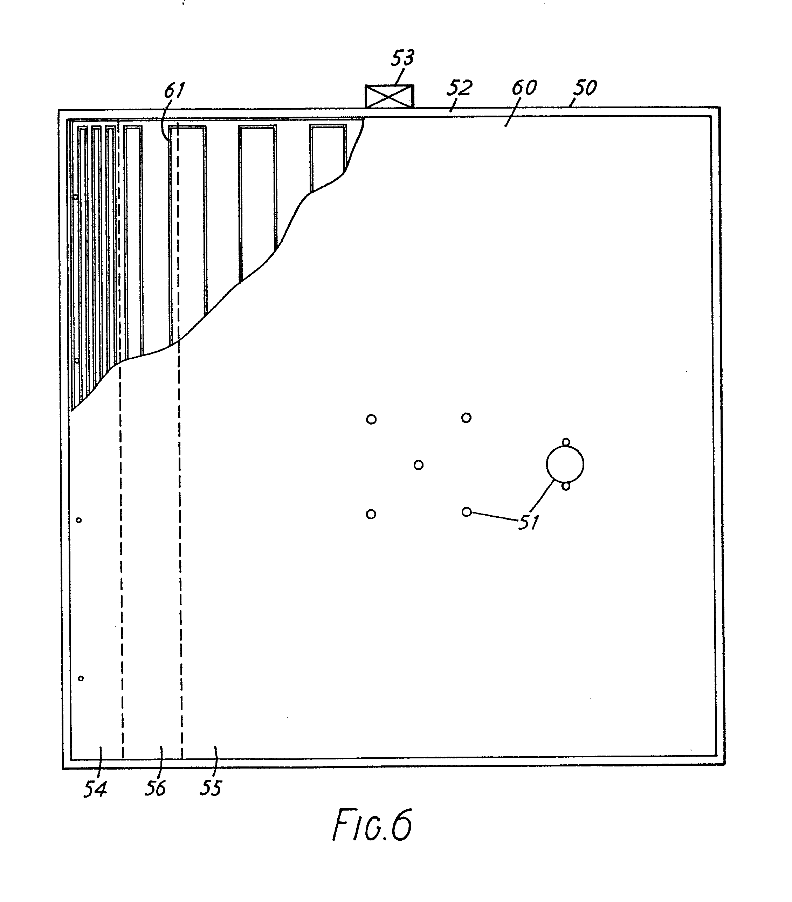

An example of a heating blanket is shown in Fig. 6.

The heating blanket 50 is made of two layers of mylar polyester 60 interleaved with a distributed foil element. The elements are connected in series to a 220/240 volt supply and consumes about 100 watts.

One side of the blanket has a self adhesive coating. The self adhesive coating is for mounting the blanket on the wall or door of the incubator. The adhesive used is stable up to at least 130°C.

Holes 51 are provided in the blanket at places which correspond to protruding supports, instruments etc. on the wall of the main cavity. The foil element extends as close as possible to the edges of the holes while maintaining a safe margin for electrical insulation.

An element-free margin 52 is also left around the edge of the blanket for electrical safety.

The element is provided with terminals 53 for connection to the power supply and controls housed in the instrument unit.

In Fig. 6 part of the top layer 60 of mylar polyester has been removed to show part of the foil element 61. The foil element consists of a strip of metallic foil which follows a meandering path across the blanket. Both ends of the strip terminate at the terminals 53. The width of the strip and/or the spacing between adjacent sections of the strip is different in different parts of the blanket so that the heat output varies across the blanket. In the embodiment the density of the foil track strip is in the narrow region 54 approximately four times the density in the adjacent narrow region 55 which in turn is about one and a half times the density in the remainder of the blanket.

Thus, in a particular example, the foil element is distributed within the blanket in the following manner:
30% of the element is distributed evenly over an area 54 of 40mm x 544mm
60% of the element is distributed evenly over an area 55 of 454mm x 544mm
10% of the element is distributed over an area 56 of 50mm x 544mm and decreasing in density from the junction with the high density area 54 towards the lower element density area 55.

The variations in foil width and/or density compensate for variations in heat loss from different parts of the incubator. The heat loss tends to be greater towards the glass inner door. The element shown in Figure 6 is designed for the four walls adjacent the glass door. The element on the rear wall is designed with a high density area closest to the fan storage where the extra heat is conducted away.

Alternatively where greater economy of manufacture is required, the same foil element may be used on all six walls of the chamber. The heating element may still be as shown in Fig. 6. The blanket is arranged on the rear wall with the side of the heating element having the more closely spaced track, located nearest the fan. Two heating elements may be mounted on the door, one with the more closely spaced track nearer the top and the other with the more closely spaced track nearest the bottom. It will be noted that the chamber is substantially cube shaped so that all walls are the same size and shaped to enable the same foil track heating element to be used on all walls.

On the inner shell walls of the housing are mounted brackets for nylon shelf spacers 33 by which runners 34 are held. Shelves 35 are supported by the runners. The shelves are stainless steel baffle plates with a rectangular array of circular holes.

On the inner surface of the roof of the housing are mounted a gas sensor ($CO_2$) 36 and a temperature sensor 37. These sensors are connected by wires to control circuits 38 on the instrument unit. A humidity reservoir 41 is provided in the base of the housing.

The instrument unit 5 mounted beside the housing, contains a control panel 39 for setting the temperature and $CO_2$ level. The control panel then displays the actual temperature and $CO_2$ levels by means of the sensors in the housing and the control circuit switches on and off the current to the heating blankets or the gas flow to achieve and maintain a stable environment.

The instrument unit also contains a gas feed 40 from an external source. This allows pure $CO_2$ to be fed into the housing.

## Claims

1. An incubator or oven comprising an insulated housing, the wall of which are lined with foil track electrical heating elements, and a temperature sensor for controlling the supply of electricity to the heating elements according to the temperature in the incubator or oven.

2. An incubator or oven according to claim 1 in which the walls of the housing comprise outer and inner shells with insulating material contained between them.

3. An incubator or oven according to claim 1 or 2 in which the housing has a door and foil track heating element is mounted on the inside of the door.

4. An incubator or oven according to any preceding claim in which the housing has a door comprising inner and outer shells with insulating material contained between the shells.

5. An incubator or oven according to claims 3 or 4 which is provided with an inner door of transparent material adjacent to the inside of the door of the housing.

6. An incubator or oven according to any of the preceding claims in which the housing is rectangular and has a door forming at least part of one wall of the housing, the other five walls of the housing all being lined with foil track heat elements.

7. An incubator or oven according to any of the preceding claims wherein the foil track pattern of heating element on at least one wall varies across the area of the heating element so that the heat generated by the foil track varies across the heating element.

8. An incubator or oven according to claim 7 in which the foil track pattern is varied by varying the density of the foil track pattern.

9. An incubator or oven according to claim 8 in which the foil track is of uniform width throughout its length.

10. An incubator or oven according to claim 7, 8 or 9 in which the same design of heating element is used on more than one wall of the incubator or oven.

FIG.1

0238313

FIG.2

FIG.3

0238313

FIG.4

0238313

FIG.5

FIG.6

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | DE-A-2 259 377 (W.C. HERAEUS) <br><br> * Claims 1,7,11,12,14; page 6, first and second paragraphs; figure 2 * | 1,2,5, 6,9,10 | C 12 M 1/00 <br> B 01 L 7/00 |
| X | US-A-3 783 238 (G.C. DIETZ et al.) <br> * Claims; figures * | 1,9,10 | |
| Y | US-A-4 572 427 (G. D. SELFRIDGE et al.) <br> * Claims 1-3; column 6, line 38 - column 7, line 11; figure 2 * | 1-10 | |
| Y | FR-A-2 365 268 (A. KOGLER) <br> * Claim 1; page 6, lines 20-33; figure 1 * | 1-10 | **TECHNICAL FIELDS SEARCHED (Int. Cl.4)** |
| A | DE-A-1 914 585 (A. FINK) <br> * Claims 1,4,6-8; figure 4 * | 1,2,5 | C 12 M <br> B 01 L <br> H 05 B |
| A | FR-A-2 132 759 (ROTAX LTD.) <br> * Claim 1; page 2, lines 11-20 * | 1,10 | |

The present search report has been drawn up for all claims

| Place of search | Date of completion of the search | Examiner |
|---|---|---|
| THE HAGUE | 17-06-1987 | WIESER, M.R.J. |